# EUROPEAN PATENT SPECIFICATION

(11) **EP 3 865 874 B1**
(45) Date of publication and mention of the grant of the patent: **05.10.2022**
(21) Application number: 21154366.5
(22) Date of filing: 29.01.2021
(51) Int. Cl.: G01N 33/558, G01N 33/92, G01N 33/94, G01N 33/52

(54) **DISTINGUISHING SMOKING E-CIGARETTES FROM SMOKING CIGARETTES**
UNTERSCHEIDUNG DES RAUCHENS VON E-ZIGARETTEN UND DES RAUCHENS VON ZIGARETTEN
DISTINGUER DES CIGARETTES ÉLECTRONIQUES ET DES CIGARETTES

(30) Priority: 13.02.2020 CN 202010090249; 11.05.2020 US 202063023213 P
(43) Date of publication of application: 18.08.2021
(73) Proprietor: Zhejiang Orient Gene Biotech Co., Ltd, Anji Huzhou Zhejiang 313300 (CN)
(72) Inventor: Shen, Lili, Anji Huzhou, Zhejiang 313300 (CN); Feng, Haiying, Anji Huzhou, Zhejiang 313300 (CN); Fang, Jianqiu, Anji Huzhou, Zhejiang 313300 (CN)
(74) Representative: Ipey

(56) References cited:
- EP-A1- 0 821 069
- WO-A1-2016/118886
- LEE YOUNG-JAE ET AL: "Quantitative insights into major constituents contained in or released by electronic cigarettes: Propylene glycol, vegetable glycerin, and nicotine", SCIENCE OF THE TOTAL ENVIRONMENT, ELSEVIER, AMSTERDAM, NL, vol. 703, 2 November 2019 (2019-11-02), XP086042263, ISSN: 0048-9697, DOI: 10.1016/J.SCITOTENV.2019.134567 [retrieved on 2019-11-02]
- CRENSHAW MICHAEL D. ET AL: "Determination of nicotine, glycerol, propylene glycol and water in electronic cigarette fluids using quantitative 1 H NMR : Electronic cigarette fluids", MAGNETIC RESONANCE IN CHEMISTRY, vol. 54, no. 11, 24 August 2016 (2016-08-24), pages 901-904, XP55794276, GB ISSN: 0749-1581, DOI: 10.1002/mrc.4498 Retrieved from the Internet: URL:https://api.wiley.com/onlinelibrary/td m/v1/articles/10.1002%2Fmrc.4498>
- ACHILIHU HONEST ET AL: "Abstract", JOURNAL OF ANALYTICAL TOXICOLOGY, vol. 43, no. 2, 3 November 2018 (2018-11-03), pages 149-153, XP55794282, US ISSN: 0146-4760, DOI: 10.1093/jat/bky075 Retrieved from the Internet: URL:http://academic.oup.com/jat/article-pd f/43/2/149/33407452/bky075.pdf>
- VOLKOV ALEXANDER ET AL: "Rapid prototyping of lateral flow assays", ANTIBODY-DRUG CONJUGATES; IN: METHODS IN MOLECULAR BIOLOGY; ISSN 1064-3745; VOL. 263; [METHODS IN MOLECULAR BIOLOGY; ISSN 1064-3745], HUMANA PRESS, US, vol. 504, 11 December 2008 (2008-12-11), pages 217-235, XP009166393, DOI: 10.1007/978-1-60327-569-9_14 ISBN: 978-1-62703-541-5 [retrieved on 2011-01-07]

## Description

### FIELD OF THE INVENTION

The invention relates to the use of a device for distinguishing a test subject smoking an e-cigarette from smoking a cigarette (i.e. a traditional cigarette).

### BACKGROUND OF THE INVENTION

Illegal abuse of drugs has become a recognized and worsening social issue in our society. In 2003, an investigation made by the United States Department of Health and Human Services found, about 19.5 million Americans, or 8.2 percent of people aged 12 and above, were taking illegal drugs. "Recent use of an illegal drug" is defined as use of an illegal drug within the month prior to the investigation made by the United States Department of Health and Human Services. According to the data, Marijuana was the most common illegal drug, which accounts for 6.2 percent (14.6 million). It is estimated that 2.3 million people (1.0%) are now taking cocaine, 604,000 are taking crack, 1 million are taking vertigo, and 119,000 are taking heroin. With the development and change of times, a large number of new drugs keep emerging, which have become one of the important social issues around the world.

To struggle against drug abuse and monitor such social issue, drug test has become a standard testing procedure in industries such as employment, education, sports, and law enforcement. To boost this effort, the drug testing industry has been formed. The industry offers a wide range of drug testing products. The urine collection cup, which can analyse a sample, is a classic testing product. These devices may be complex, difficult to operate for users; or if the sample is leaked, it will cause contamination to people; or the device is properly operated, it will not identify the real drug users. Besides, urine samples cannot be collected in certain cases, such as at roadside or public places.

Collecting liquid samples (such as urine) in detection devices and judging if there is presence of any specific analytes (such as drugs and/or their metabolites, or disease-related labelling substances), has become a common method. A device for such test generally requires that the sample be collected in a sample container, the technician inserts a test strip in the container (with part of the strip submerged in the sample), and then takes the strip out and reads the result. The technician may have contact with the sample, which may endanger his/her health or contaminate the sample. To avoid such risk, the sample collection container can only be operated after capping a closed cover. At present, there are many well-closed devices, including those disclosed by US patents US 4,976,923, US 5,429,804, and US 6,726,879. These devices fix the test strip on the cover of the detection device. When in use, the container may be turned over or tilted to make the test strip submerged in the sample for testing. The US patent application US2003/0027359A1, disclosed on February 6, 2003, reveals a urine testing cup. After the cover of the urine testing cup is capped to the opening of the cup, a push rod is used to push the plunger to move and make the fluid sample flow out of the cup chamber and moisten the testing element. The patent application 200510113977.5 disclosed in China, reveals a urine testing cup. After the cover of the urine testing cup is capped to the opening of the cup, the fluid starts to flow from the collection chamber to the testing chamber. In this way, the testing process is started. Another patent application 200480033286.8 disclosed by China, also revealed a urine testing cup. The urine testing cup starts to test when the cover is capped to the opening of the cup.

In drug abuse, nicotine is one of them. Nicotine is an important component in tobacco. Both active smoking and passive smoking can have adverse effects on the human body, for example, cardiovascular disease, chronic obstructive pulmonary disease and even cancer. Tobacco addiction has now become a prominent threat to public health in the society. The main metabolites of nicotine in human body are cotinine and 3-hydroxycotinine; and cotinine accounts for 70% to 80% of all metabolites. As reported, the concentration of nicotine and its metabolites in different body fluids (blood, urine, and saliva) is closely related to the amount of smoke exposure, and its level can be used to assess smoking, smoking cessation behaviour, as well as environmental smoke exposure. Therefore, it is important to develop a fast and accurate method for simultaneously detecting nicotine and its metabolites.

The main source of nicotine is cigarettes made from tobacco leaves. At the same time, with the popularity of electronic products, there are also electronic cigarettes for smoking. Regardless of different forms, the main ingredient is nicotine.

This requires a product to detect whether nicotine is taken, for example from cigarettes or e-cigarettes.

Achilihu Honest ET AL: "Abstract", Journal of Analytical Toxicology, vol. 43, no. 2, 3 November 2018 (2018-11-03), pages 149-153, XP55794282, US ISSN: 0146-4760, DOI: 10.1093/jat/bky075 discloses a method of classifying the use of tobacco using urinary cotinine (lateral flow) immunoassay test strips.

### SUMMARY OF THE INVENTION

The present invention comprises the use of a device for distinguishing a test subject smoking an e-cigarette from smoking a cigarette (i.e. a traditional cigarette), characterised in that,
the device comprises a test strip adapted to detecting nicotine in a sample; and a test strip adapted to detecting glycerine in the sample;
if the test strip for detecting nicotine is positive and the test strip for detecting glycerine is negative, it means the test subject smokes cigarettes ;
if the test strip for detecting nicotine is positive and the test strip for detecting glycerine is positive , it means the test subject smokes both cigarettes and e- cigarettes;
if the test strip for detecting nicotine is negative and the test strip for detecting glycerine is positive, it means the test subject smokes e-cigarettes, wherein the sample is used for detecting the nicotine and the glycerine.

It is to be understood that the invention generally relates to the aforementioned use. Embodiments listed below relate to further limitations of said use.

The test strip adapted to detecting the nicotine may be a lateral flow test strip.

The lateral flow test strip may detect the nicotine by immunoasssay.

The test strip may comprise antibodies and antigen analogues for detecting cotinine and 3-hvdroxycotinine.

The antibody may be combined with a labelling substance and the antibody-labelling substance can be removed.

The test strip may comprise cotinine with BSA fixed to the test strip.

The test strip adapted to detecting glycerine may comprise a reagent to detect the glycerine, and the reagent comprises a colour development substrate and an enzyme.

The enzyme may be one or several of glycerol kinase, glycerol-phosphate oxidase or peroxidase.

The colour development substrate may be one or more of 3,3,5,5 - tetramethyl benzidine, 3,3,5,5 - tetramethyl benzidine hydrochloride, 4, 3,5-dichloro-2-hydroxybenzene sulfonic acid sodium, N, N - dimethyl aniline, N, N - dimethyl aniline dihydrochloride, N- (2 - hydroxy - 3 - sulfonated propyl) - 3,5 - dimethoxy aniline sodium, and 2,4,6-three bromine - 3-hydroxy benzoic acid.

The reagent may further comprise a buffer reagent and a stabilizing reagent.

The stabilizing reagent may be one or several of bovine serum albumin, polyethylene glycol 2000, polyvinyl pyrrolidone, or ethyl cellulose.

The sample may be saliva, blood and urine.

The sample may be saliva.

### DETAILED DESCRIPTION

The structure and the technical terms used in the invention are explained as below. The explanations merely take examples to illustrate how the use of the invention is realized and do not constitute any limitation on the invention. The scope of the invention is limited and expressed by the claims.

### TEST

A test is to conduct experiment or test to determine the presence of a substance or material, for example, but not limited to, chemicals, organic compounds, inorganic compounds, metabolic products, drugs or drug metabolites, organic tissue or metabolites of organic tissues, nucleic acids, proteins, or polymers. In addition, a test indicates the quantity or presence of a substance or material. Furthermore, test also means immunity test, chemical test, enzyme test, nucleic acid test, etc.

### DOWNSTRAM OR UPSTREAM

Downstream or upstream is defined by the direction in which the liquid flows, and generally liquid flows from upstream to downstream. Liquid in the downstream region or received from the upstream region: the liquid can also flow along the upstream region to the downstream region. It is generally defined by the direction in which the liquid flows. For example, in some materials where capillary force is used to make the liquid flow, the liquid can flow by gravity in the opposite direction to gravity. In this case, the upstream and downstream of the liquid are also defined by the direction in which the liquid flows.

### GAS FLOW OR LIQUID FLOW

Gas flow or liquid flow refers to liquid or air's ability to flow from one place to another, and the process of flow may be guided by some physical structures. The flow here can be liquid flow or gas flow, and it can be passive flow due to its own effect (gravity or pressure).

### A TEST ELEMENT FOR TESTING NICOTINE

Combination of a variety of test elements can be applied to the invention. The testing element includes test strip, which can be analysed in various forms, such as immunological or chemical test. It is used to detect analytes in the sample, including drugs or metabolites that indicate the physical condition. In some embodiments, the test strip refers to the absorbent material having a liquid sample feeding area, a reagent area, and a test result area. The sample is added to the feeding area and flows into the reagent area under the action of capillary. In the reagent area, the sample dissolves the reagent and mixed with the reagent to test the analyte (if there is any analyte in the sample). Definitely, the reagent area and the sample feeding area can be the same area, and some reagents used to treat liquid samples are pre-treated in the sample feeding area. At this moment, the sample with reagent continues to flow to the test result area. Additional reagents are fixed in the test result area. The reagents fixed in the testing area react and combine with the analyte (if any) or with the first reagent in the reagent area. In the non-competitive testing mode, a signal will be generated if there is any analyte in the sample; and no signal will be generated if there is no analyte. In the competitive testing mode, a signal will be generated if there is no analyte in the sample; and no signal will be generated if there is any analyte. The invention is applicable to the testing elements with various analysing modes.

When the testing element is a test strip, it can be made of water-absorbent or non-absorbent materials. A test strip can use several materials to transfer liquid. One test strip material can be superimposed on another test strip material; for example, filter paper is superimposed on nitrocellulose. Alternatively, one area in the test strip containing at least one material is located behind another area containing at least a different kind of material. In this case, the liquids flow between the areas, and they can be superimposed or not superimposed on each other. The material on the test strip can be fixed on, for example, the support of plastic liners or a hard surface, to improve the holding power of the test strip.

In some embodiments where the tested object is detected by the signal generation system (e.g., at least one enzyme specifically reacts with the tested object), at least one signal generation substance can be adsorbed to the analyte detection area of the test strip as specifically adsorbed to the material of the test strip as described above. In addition, the signal generation substances that exist in the feeding area, reagent area, analyte detection area, or throughout the test strip can be pre-treated on one or more materials of the test strip. This can be achieved by applying the solution of signal generation substance to the surface of the application area or by immersing one or more materials of the test strip in the signal solution. After adding the signal solution to the test strip or immersing in the solution, dry the test strip up. In addition, in this method, the signal generation substances that exist in the feeding area, reagent area, analyte detection area, or throughout the test strip can be pre-treated on one or more materials of the test strip. In addition, the signal substance existing in the sample area, reagent area, or detection area of the test strip can be added to one or more surfaces of the test strip material as labelling reagent.

Areas of the test strip can be arranged as follows: a complete and necessary test strip can include a sample application area and a testing area. Generally, the liquid first contacts the sample application area, and then flows to the testing area based on capillary effect. Definitely, the test strip can also include the following areas as needed, a sample feeding area or sample application area, or at least one reagent area, or at least one testing area. The testing area includes a test result area, or also includes at least one control area, or may include at least one adulteration detection area and liquid absorption area. If the detection area includes a control area, the control area is preferred to locate behind the analyte detection area in the test result area. All of the areas or the combinations can be on a single test strip containing one material. In addition, these areas are made of different materials and are connected together in the liquid transfer direction. For example, different areas can transfer liquid directly or indirectly. In this embodiment, different areas can be connected by end to end along the direction of liquid transfer or superimposed on each other along the direction of liquid transfer, or connected through other materials, e.g., connecting media materials (preferably water-absorbing materials such as filter paper, glass fibre or nitrocellulose). When using connecting material, the connecting material can make the end containing all areas and the material connecting to the end; the end containing all areas and the material connecting to the end but without liquid flow, material containing overlapped areas ((including but not limited to overlapping from the starting point to the end) but without liquid achieve liquid flow.

If the test strip includes an adulteration detection control area, this area can be placed before or after the result detection area. When the result detection area includes a control area, the adulteration control area is preferably placed before the control area, or otherwise. According to an embodiment of the invention, the test strip is a control test strip for adulteration analysis judgment and/or control. The adulteration control area can be located before or after the control area, and it is preferred to locate before the control area.

The commonly used reagent strip is a nitrocellulose membrane reagent strip. The detection area includes a nitrocellulose membrane, and specific binding molecules are fixed on the nitrocellulose membrane to indicate the detection result; it can also be a cellulose acetate membrane or a nylon membrane. For example, the test strip or device containing a test strip of the following patents: US 4857453; US 5073484; US 5119831; US 5185127; US 5275785; US 5416000; US 5504013; US 5602040; US 5622871; US 5654162; US 5656503; US 5686315; US 5766961; US 5770460; US 5916815; US 5976895; US 6248598; US 6140136; US 6187269; US 6187598; US 6228660; US 6235241; US 6306642; US 6352862; US 6372515; US 6379620; and US 6403383. The test strips disclosed in the above patent documents and the similar devices with a test strip can be applied to the testing element or testing device of the use of the invention for detecting analyte, for example the detection of a divided substance from the sample.

In a solution, a similar substance with nicotine is fixed on the test strip, an antibody against nicotine or a similar substance is treated at upstream of the test area, and the antibody carries a labelling substance. The labelling substance can be coloured particles, such as latex or metal particles, or fluorescent.

### TEST ELEMENT FOR TESTING GLYCEROL

In some embodiments, the test area of the test strip used to detect glycerol includes the reagent that allows glycerol to develop a colour change. These reagents generally include enzymes and white substrates. In some embodiments, a buffer agent having a buffering effect and a protective agent are also included. The material of the reagent strip includes fibre filter paper, cotton cloth and other water-absorbent materials. These water-absorbent materials can be bonded onto the non-absorbent materials. When contacting the sample, the liquid flows on the test strip under the capillary effect and changes colour. In some embodiments, the upstream of the test area may be a sample application area, and the sample flows through the application area to the test area, and then a chemical change occurs to detect the presence of glycerol.

The general reaction principle is that: glycerol produces peroxide under the effect of glycerine kinase and glycerol phosphate oxidase, and then produces oxygen under the effect of peroxidase, which causes the redox indicator in the reagent to change colour. The degree of the colour change indicates the amount of glycerol in the sample.

In some embodiments, the buffer reagent can be one or several of the following, phosphate buffer system, GOODS buffer system, the best GOODS buffer system. In the preferred embodiments, MOPSO and PIPES systems are the most suitable. In some embodiments, the buffer concentration is between 20mmol/L and 1mol/L; the optimal is between 0.1mol/L and 0.5mol/L.

In some embodiments, enzyme can be one or several of the following: glycerol kinase 1KU-1000KU/L, preferably 10-20KU/L; glycerol phosphooxidase 1KU-1000KU/L, preferably 5-40KU/L; or peroxidase 1KU-1000KU, preferably 100-200KU/L.

In some embodiments, redox indicators can be one or several of the following: including but not limited to, Trinders reagent, 3,3,5,5 - tetramethyl benzidine, 3,3,5,5 - tetramethyl benzidine hydrochloride, 4 - amino anti than Lin, TOOS, 3,5-dichloro-2-hydroxybenzene sulfonic acid sodium, N, N - dimethyl aniline, N, N - dimethylaniline dihydrochloride, N- (2 - hydroxy - 3 - sulfonated propyl) - 3,5 - dimethoxy aniline sodium, and 2,4,6-three bromine - 3-hydroxy benzoic acid. In some embodiments, the amount of chromogenic agent is 0.2-5g/L, preferably 0.5-2g/L.

Because the reagent contains a variety of enzymes, and the enzyme is a biological agent with unstable nature. To ensure our products can keep stable at room temperature for 2 years, we must add an appropriate stabilizer. The stabilizers to be added are included in the following. Bovine serum albumin 0.5-10g/L. In some embodiments, the protective agent can be one or several of the following and the preferred amount is 2-5g/L; polyethylene glycol 2000 0.5-10g/L, preferably 2-5g/L; polyvinylpyrrolidone 1-20g/L, preferably 5-10g/L; ethyl cellulose 0.5-10g/L, preferably 1-5g/L.

The liquid reagent prepared above is immersed in the carrier, dried, cut to the specified size and pasted on the substrate. The carrier may be fibre filter paper, cotton cloth, and other water-absorbing materials. During preparation, the medicinal solution is immersed in the water-absorbing material, dried at 50-80° C temperature, and tested by a moisture analyser after drying. The moisture content is not greater than 8%.

### SAMPLE

Samples that can be detected by the detection device of the present invention include biological fluid (such as case fluid or clinical samples). Liquid samples or fluid samples can be derived from solid or semi-solid samples, including faeces, biological tissues and food samples. The solid or semi-solid samples can be converted into the liquid samples by any suitable method, such as mixing, mashing, macerating, incubating, dissolving or utilizing enzymolysis to digest the solid samples in suitable solutions (such as water, a phosphate solution or other buffer solutions). "Biological samples" include animal, plant and food samples, for example, including urine, saliva, blood and its components, spinal fluid, vaginal secretions, sperms, faeces, sweat, secretions, tissues, organs, tumours, cultures of the tissues and the organs, cell cultures and media derived from humans or animals. A preferable biological sample is the urine. The food samples include food processing substances, final products, meat, cheese, wine, milk and drinking water. The plant samples include any plants, plant tissues, plant cell cultures and media. "Environmental samples" are derived from environment (for example, the liquid samples from lakes or other water bodies, sewage samples, soil samples, groundwater, seawater and waste liquid samples). The environmental samples may also include sewage or other wastewater.

Any analyte can be detected by utilizing the present invention and a suitable detection element. The present invention is preferably utilized to detect small drug molecules in the saliva and the urine. The small drug molecules can be cotinine and 3-hydroxycotinine.

### ANALYTE

The analytes detected in the invention are glycerine and nicotine. Examples (not claimed) that can use the analyte include some hapten substances which include drugs (such as drug of abuse). "Drug of abuse" (DOA) refers to use of drugs for non-medical purposes (usually paralyzing nerves). Abuse of these drugs can lead to physical and mental damage, causing dependence, addiction and/or death. Examples of DOA include cocaine; amphetamine (AMP) (such as black beauty, white amphetamine tablets, dexamphetamine, dextroamphetamine tablets and Beans); methamphetamine (MET) (crank, meth, crystal and speed); barbiturate (BAR) (such as Valium□, Roche Pharmaceuticals, Nutley and New Jersey); sedatives (i.e. sleeping aids); lysergic acid diethylamide (LSD); inhibitors (downers, goofballs, barbs, blue devils, yellow jackets and methaqualone); tricyclic antidepressants (TCA, i.e. imipramine, amitriptyline and doxepin); methylenedioxymethamphetamine (MDMA); phencyclidine (PCP); tetrahydrocannabinol (THC, pot, dope, hash, weed, etc.); opiate (i.e. morphine (MOP) or opium, cocaine (COC), heroin and hydroxycodeinone); and antianxietics and sedative hypnotics, wherein antianxietics are a class of drugs mainly used for reducing anxiety, tension and fear, stabilizing mood and having hypnotic and sedative effects, including benzodiazepines (BZO), atypical BZ, fusion diazepines NB23C, benzodiazepines, BZ receptor ligands, ring opening BZ, diphenylmethane derivatives, piperazine carboxylates, piperidine carboxylates, quinazolinones, thiazines and thiazole derivatives, other heterocyclics, imidazole sedatives/paregorics (such as oxycodone (OXY) and methadone (MTD)), propylene glycol derivatives-carbamates, aliphatic compounds, anthracene derivatives, etc. The detection device can also be used for detecting drugs that belong to medical use but are prone to overdose, such as tricyclic antidepressants (imipramine or the like) and acetaminophen. After being absorbed by the human body, these drugs will be decomposed into different small molecule substances which are present in body fluids such as blood, urine, saliva, sweat or part of the body fluids.

### DETECTION DEVICE

The detection device can detect presence or quantity of the analyte in a sample using any technical principle, i.e., achieving qualitative or quantitative detection. The detection device includes a test element for detecting presence or amount of the analyte in the sample. In addition to the test element, the detection device further includes a device for containing the test element.

For example, an immunological test strip is provided to detect nicotine or its metabolites in the saliva sample, while another test strip is provided to detect glycerol in the same saliva. After the sample can be collected, the liquid sample contacts the two test strips to complete reaction.

Of course, these two test strips can still be arranged in a container which is used for collecting a fluid sample, such as the saliva or the urine. After the fluid sample is collected, the two test strips can contact the same sample simultaneously to complete testing. In the specification of the prevention invention, including the abstract and the claims, the "a" should be understood as being at least one, or one in number, and should not be understood as "unique" or "only one". The two test strips can also be applied to other detection devices, in particular to those devices that include a cover and adopt the cover to seal an opening of a collection cavity; and such other similar devices are specifically described in disclosed United States Patents US 7,270,959, US 7,300,633, US 7,560,272, US 7,438,852, US 4,976,923, US 5,429,804 and US 6,726,879. The detection device disclosed in the present invention can be combined into specific embodiments of each detection device disclosed by the above patents to serve as an embodiment in the use of the present invention.

### E-CIGARETTE

E-liquid, also known as nicotine liquid, is electronic atomizing liquid matched with the e-cigarette in use. It can produce mist like cigarettes by means of heating by an e-cigarette atomizer. Chinese name: e-liquid, foreign name: Electronic Cigarette Liquid/E-Liquid, nickname: nicotine liquid. Main raw materials: glycerine, tobacco flavour and tobacco extract. Main components of the e-liquid include edible or pharmaceutical grade glycerol, propylene glycol, polyethylene glycol and special tobacco flavour. Some e-liquid also contains nicotinamide, mainly for making taste closer to that of cigarettes.

Ingredients in the e-liquid: nicotine salt (addictive substance), PG/VG (respectively for flavour carrying and fogging), flavour and fragrance (common). Among them, Food and Drug Administration (FDA) defines PG as "general safe to the human body"; VG is also known as vegetable glycerine and is widely used in food and cosmetics. The flavour is common and has no effect on the human body. The standard e-liquid is composed of edible propylene glycol, glycerine, nicotinamide, etc. Nicotinamide: Actually, it refers to nicotine. In order to make the e-liquid smoke more similar to real cigarettes, many e-liquids has nicotine added in different concentrations, and consumers can choose whether they need the nicotinamide and nicotinamide concentration.

In the present invention, the glycerine is selected as a test subject. According to several tests, the glycerine has the best effect and is high in product sensitivity. When a chemical method is adopted to detect other substances, such as glycerol, propylene glycol and polyethylene glycol, a detection effect is not very good, and it is difficult to detect by a conventional chemical method. Meanwhile, although the method can be used, test missing is easily caused, and this may be caused by the fact that the glycerol, the propylene glycol and the polyethylene glycol are low in the samples (the saliva or the urine) and has volatility.

The e-cigarette is an imitated cigarette electronic product having the same appearance, smoke, taste and feel as the cigarette. It is a product for a user to smoke by Atomizing turning Nicotine the nicotine into steam by means of atomizing. The nicotine here is mainly extracted from the plants and then artificially synthesized.

Although there are different styles or brands of e-cigarettes, the e-cigarette is generally mainly composed of 3 parts of a cigarette tube containing a nicotine solution, an evaporation device and a battery. An atomizer is powered by a battery rod and can transform the liquid nicotine in the tobacco tank into the mist, so that the user has a smoking-like feeling when inhaling, and the effect of "puffing" can be achieved. Spices in various taste such as chocolate and mint can be added to the cigarette tube according to personal preference. In some ways, the e-cigarette includes glycerine,

Cigarette rod. An internal structure of the cigarette rod adopts same basic components: a lamp PCBA board, a rechargeable battery and various electronic circuits. Most e-cigarettes use lithium-ion and secondary battery power components. Battery life depends on battery type and size, number of uses and operating environments. Many different types of battery chargers are available, such as direct chargers, car chargers and USB interface chargers. The battery is the largest component of the e-cigarette. Some e-cigarettes adopt an electronic airflow sensor to activate a heating element, and a battery circuit works as soon as the user inhales. For manual sensing cigarettes, the users need to press a button before smoking. Pneumatic cigarettes are convenient to use, manual cigarettes are relatively stable than the pneumatic cigarettes and have better smoke output. With development of hardware and software, some manufacturers have begun to develop fully automatic mechanical manufacturing of the e-cigarette, eliminating manual wiring, soldering or electronic work to achieve higher safety and reliability.

Atomizer. Generally speaking, a tobacco tank is a mouthpiece part, but some factories glue the atomizer with the tobacco tank or the e-liquid to make a disposable atomizer according to customer demands. The advantages are that the taste and smoke quantity of the e-cigarette can be greatly improved, and the quality is more stable at the same time. Because the atomizer is the most easy to break, the conventional e-cigarette is a separate atomizer which breaks down in a few days. Due to the fact that factory professionals fill the liquid, the problem that the tobacco liquid flows into a mouthpiece or flows to another battery part and causes corrosion to the circuit during manual filling is solved, the e-liquid quantity is also more than that of common tobacco tanks, a sealing performance is good, and a service life is longer than that of other tobacco tanks accordingly.

This technology is only owned by very few brands. A structure of the atomizer is a heating component which heats under power of the battery to make the e-liquid beside volatile to form the smoke, so that the effect of "puffing" can be achieved when the user inhales.

### TOBACCO PRODUCTS

The tobacco referred to here is a concept relative to the e-cigarette. Cigarette products are processed from the plants containing the nicotine, and the tobacco () is used most. Plant tobacco is processed from the plants containing the nicotine. Generally, there are two varieties of the tobacco that can be smoked; one is ordinary tobacco, also known as tabacum which is an annual, biennial or triennial herbaceous plant. This tobacco should be preferably grown in warmer zones. Another is makhorka, also known as nicotiana rustica which is an annual or biennial herbaceous plant. This tobacco has high freeze resistance capability and is suitable to be cultivated in low temperature areas. In addition, there is also a kind of whiteflower tobacco cultivated by Chileans, with attractive green leaves and white flowers.

The tobacco can be divided into many varieties, and there are five major categories in the United States currently. According to variety characteristics, cultivation conditions, preparation methods and main purposes, various tobaccos cultivated from ancient times in China is mainly divided into the following 6 categories: Sun-cured tobacco: This variety has a wide production area and the longest history of planting, almost all over the world. This the first tobacco variety introduced into China. The tobacco found in the beginning is like makhorka, collectively called sun-cured tobacco, commonly known as Chinese prepared tobacco. A method for preparing the tobacco products is also simple. The method generally includes picking mature tobacco leaves grown in a field, tying the leaves, suspending the leaves under eaves for airing and drying to form the tobacco leaves, then making the tobacco leaves into the cigar tobacco and cut tobacco by hand and smoking them by using a simple smoking set. There are two ways of production and consumption: one is self sufficiency for farmers, or with a small amount sold; another is large-scale production of the sun-cured tobacco for manufacturing the tobacco products, such as the cigar tobacco, the cut tobacco, snuff and chewing tobacco. The sun-cured tobacco can also be used for producing the cigarettes in small quantities. However, it is strong in spicy taste, high in irritation and narrow in consumption range. After research and trial planting, many high-quality tobacco leaf varieties are successfully cultivated. At the same time, the original sun-cured tobacco quality is improved, and unique local sun-cured tobacco is formed. Flue-cured tobacco: It is native to Virginia, USA, called Virginia-type flue-cured tobacco internationally, and also called American tobacco. Because this kind of tobacco leaves is heated and baked by a fire tube in a flue-curing barn, so it is called flue-cured tobacco. After the tobacco leaves are baked, the leaves are golden in colour, bright in lustre and mellow in flavour, so they are the main raw material for producing the cigarettes in various countries in the world. Its yield accounts for about more than 40% of the global tobacco output. The main raw materials of flue-cured tobacco cigarettes are the flue-cured tobacco, and the flue-cured tobacco is also used in production of other types of the tobacco products. Main producing countries of the flue-cured tobacco include China, the United States, Canada, India, Zimbabwe, etc. The output of the flue-cured tobacco in China accounts for more than 80% of the total output of the tobacco leaves. The production of the flue-cured tobacco is mainly concentrated in Yunnan, Henan, Guizhou, Shandong, etc. Burley tobacco: It is native to the United States. The burley tobacco is named because its milky white stems and veins. It is a kind of dark brown sun-cured tobacco. A preparation method includes building an airing gate that can control temperature and humidity and suspending the mature tobacco leaves in the gate for preparation and airing. This kind of tobacco leaves has a strong aroma and high nicotine content and is the main raw material for producing mixed cigarettes. Countries for planting the burley tobacco include the United States, Brazil, Japan, etc. From 1956 to 1966, China successively planted the burley tobacco in Shandong, Henan, Anhui, etc. Since the 1980s, the burley tobacco has been planted in Hubei and Chongqing. With improvement of the tobacco leaf quality, the burley tobacco has been utilized to produce the mixed cigarettes. Aromatic tobacco: It is mainly produced in Turkey, Bulgaria, Greece, Thailand, etc. It is a special variety with small leaves that contain high aromatic substances. It is compound tobacco for producing the mixed cigarettes and can also be adopted to produce oriental-type cigarettes with increased dosage. This type of cigarette is produced in Eastern European countries such as the former Soviet Union and Bulgaria. But aromatic tobacco leaves are low in yield, generally 40-50 kg per mu, so a selling price is high, and only a small amount can be utilized to produce the mixed cigarettes. Global production of this tobacco is not high. Cigar tobacco: This does not mean finished cigars rolled but refers to a raw material for making the cigars -- the cigar tobacco as tobacco leaves. The raw tobacco leaves for making the cigars have very high requirements and are divided into three types of packed-leaf tobacco, bundled-leaf tobacco and core-leaf tobacco. Among them, the packed-leaf tobacco has the most stringent requirement which requires thin light leaves, thin veins, fine tissues, high elasticity and tension and uniform glossy colour. This packed-leaf tobacco is usually specially cultivated and better cultivated in shade. It is air-dried in a room after picked and belongs to the sun-cured tobacco. A main producing area of the packed-leaf tobacco in China is Sichuan while the packed-leaf tobacco produced in Tongxiang, Zhejiang has the best quality. Many sun-cured tabacum produced in China can be used as the raw material for cigar bundle leaves and core leaves. Makhorka: It is different from the above 5 types of tabacum in plant classification, so there is a big difference. Its plant is shorter than that of the tabacum, and it has a short growth period and high freeze resistance. Therefore, all the makhorka in China is grown in the north. Among them, the famous ones are Lanzhou makhorka (Lanzhou hookah), Northeast toad tobacco, Xinjiang Yili Mohe tobacco (also known as makhorka). Most are processed into pipe tobacco and the hookah. In addition, Chinese agronomists have successfully researched and selected novel \"medicine tobacco\" in recent years, and the novel medicine tobacco is cultivated by using distant hybridization between medicinal plants and the tobacco. For example, a medicinal tobacco breeding research team of Shanxi Agricultural University has successfully cultivated 9 kinds of "medicine tobacco" such as ginseng, astragalus, honeysuckle and mint containing Chinese herbal medicine ingredients. These kinds of \"medicine tobacco\" can reduce the content of harmful substances in the cigarette and have a certain curative effect on certain diseases of the human body, thereby being the promising novel tobacco.

In any case, the above plant tobacco contains the nicotine which is a component in the plant. Although it has the same structure as that of the e-cigarette, sources are different.

This not only refers to the nicotine in the tobacco, but also includes products for smoking and made from other plants containing the nicotine. For example, in addition to the tobacco, there are many varieties of nightshades that are loved by humans and cultivated in large quantities, such as eggplants, tomatoes and sweet peppers, favourite condiment pepper, potatoes used as staple food by Europeans and wolfberry known for its health benefits, all these belong to the nightshades and contain the nicotine. Calculated by dry weight, nicotine content in a potato peel can be up to 14.80 mg/kg while the content in peeled potatoes is below a detection limit (1 mg/kg). An average content in tomato fruit is 2.31 mg/kg, 2.65 mg/kg in eggplants and 3.15 mg/kg in green peppers. Since data is calculated by the dry weight, and a moisture content of the tomatoes is nearly 95%, an actual content should be obtained by dividing the data by 20, which is 0.12 mg/kg. Cooked tomato products and potato products are low in nicotine content which is basically negligible.

The detection device can distinguish between the tobacco products and the e-cigarette sensitively. A main purpose of the detection device is to distinguish different types of smoking, which has the following meanings. The first is to know that although they both contain the smoking nicotine, other toxic substances in the e-cigarette and the tobacco products are different. In this way, different medical schemes are adopted for treating or intervening nicotine poisoning, and the schemes are more accurate and targeted.

### DETECTION METHOD

The invention involves detecting an analyte in a sample. A detection device includes a test strip for detecting nicotine in saliva and a test strip for detecting glycerol in saliva. This includes making the two strips contact saliva samples and making the saliva samples flow on the two test strips respectively. If the test strip for detecting the nicotine shows positive and the test strip for detecting the glycerine shows negative, it means a test subject smokes tobacco products but does not smoke e-cigarettes;

If the test strip for detecting the nicotine shows positive and the test strip for detecting the glycerine shows positive, it means the test subject smokes both tobacco and e-cigarettes;

If the test strip for detecting the nicotine shows negative and the test strip for detecting the glycerine shows positive, it means the test subject smokes e-cigarettes but does not smoke tobacco.

### EXAMPLES

The following describes how the present invention is implemented through embodiments.

### EXAMPLE 1

Reagent preparation: Phosphate buffer system 100mM 1L PH7.5
Glycerin kinase 10KU
Glycerol phosphate oxidase 20KU
Peroxidase 20KU
3, 3, 5, 5-TMB HCL 1g
PVP 2g
Bovine serum albumin 5g

Immersing and drying: Use GE's 3MM filter paper, immerse it in the saliva and dry at 50°C for use. The saliva containing glycerine and cotinine was tested, and the test paper developed from colourless to blue. The lowest threshold that can be detected was 5000 ng/mL.

Providing a test strip in an immunity test sample in the prior art: The test strip includes a sample application pad which is superimposed on a label pad, the label pad is provided with an anti-cotinine antibody labelled with gold particles, the label pad is connected with a detection pad made of a nitrocellulose film, and the detection pad is provided with the cotinine with BSA.

### EXAMPLE 2

Reagent preparation MOPSO buffer system 100mM 1L PH7.0
Glycerin kinase 10KU
Glycerol phosphate oxidase 20KU
Peroxidase 20KU
Amino-antipyrine 0.5g
TOOS 1g
PVP 2g
Bovine serum albumin 5g

Immersing and drying: Use GE's 3MM filter paper, immerse it in the saliva and dry at 50°C for use. A sample containing the glycerol was tested, and the test paper developed from colourless to pink or purple. The lowest threshold that can be detected was 4000ng/mL.

Colloidal gold COT: Providing a test strip in an immunity test sample in the prior art: The test strip includes a sample application pad which is superimposed on a label pad, the label pad is provided with an anti-cotinine antibody labelled with gold particles, the label pad is connected with a detection pad made of a nitrocellulose film, and the detection pad is provided with the cotinine with BSA.

### COMBINATION THE TWO TESTS

Paste the prepared strips (Embodiment 1 and Embodiment 2) on a plastic substrate made of polyvinyl chloride and polyester through a special process cut the strips into strips with a width of about 3mm-5mm and place the strips into a plastic joint card for testing saliva samples from people who smoke the e-cigarette. At the same time, assemble a colloidal gold COT reagent strip in the joint card and adopt the two test strips for joint testing of the saliva samples from the people who smoke the e-cigarette.

Judging indicators: E-cigarette: colloidal gold COT shows positive (positive: C line visible, T line invisible) and a colour block of the biochemical test strip changes from yellow to pink.

Common cigarette: colloidal gold COT shows positive (positive: C line visible, T line invisible) and the colour block of the biochemical test strip does not change colour.

Results (EXAMPLE 2):

| Sample ID | Glycerin test paper (originally yellow) | COT colloidal gold test strip | Preliminary judgment |
|---|---|---|---|
| 1 | Pink | + | E-cigarette |
| 2 | No colour change | + | Common cigarette |
| 3 | No colour change | + | Common cigarette |
| 4 | Pink | + | E-cigarette |
| 5 | Pink | + | E-cigarette |
| 6 | No colour change | + | Common cigarette |
| 7 | Pink | + | E-cigarette |
| 8 | Pink | + | E-cigarette |
| 9 | No colour change | + | Common cigarette |
| 10 | Pink | + | E-cigarette |

Results (Test of EXAMPLE 1):

| Sample ID | Glycerin test paper (originally yellow) | COT colloidal gold test strip | Preliminary judgment |
|---|---|---|---|
| 1 | Blue | + | E-cigarette |
| 2 | No colour change | + | Common cigarette |
| 3 | No colour change | + | Common cigarette |
| 4 | Blue | + | E-cigarette |
| 5 | Blue | + | E-cigarette |
| 6 | No colour change | + | Common cigarette |
| 7 | Blue | + | E-cigarette |
| 8 | Blue | + | E-cigarette |
| 9 | No colour change | + | Common cigarette |
| 10 | Blue | + | E-cigarette |

Then the same method was used for clinical tests, the samples showing positive in the saliva test were subjected to the e-cigarette test. Among 150 saliva-positive samples, 56 samples were glycerol-positive. After confirmation, 55 samples smoked e-cigarettes among the 56 samples, and the test accuracy was 98%. According to a sensitivity test, a minimum detection threshold for glycerol detection of the present invention was 5000 ng/mL.

## Claims

1. Use of a device for distinguishing a test subject smoking an e-cigarette from smoking a cigarette (i.e. a traditional cigarette), **characterised in that**,
the device comprises a test strip adapted to detecting nicotine in a sample; and a test strip adapted to detecting glycerine in the sample;
if the test strip for detecting nicotine is positive and the test strip for detecting glycerine is negative, it means the test subject smokes cigarettes ;
if the test strip for detecting nicotine is positive and the test strip for detecting glycerine is positive , it means the test subject smokes both cigarettes and e- cigarettes;
if the test strip for detecting nicotine is negative and the test strip for detecting glycerine is positive, it means the test subject smokes e-cigarettes, wherein the sample is used for detecting the nicotine and the glycerine.

2. The use
according to claim 1, wherein the test strip adapted to detecting the nicotine is a lateral flow test strip.

3. The use according to claim 2, wherein the lateral flow test strip detects the nicotine by immunoasssay.

4. The use according to claim 3, wherein the test strip comprises antibodies and antigen analogues for detecting cotinine and 3-hvdroxycotinine.

5. The use according to claim 4, wherein the antibody is combined with a labelling substance and the antibody-labelling substance can be removed.

6. The use according to claim 5, wherein the test strip comprises cotinine with BSA fixed to the test strip.

7. The use according to claim 1, wherein the test strip adapted to detecting glycerine comprises a reagent to detect the glycerine, and the reagent comprises a colour development substrate and an enzyme.

8. The use according to claim 7, wherein the enzyme is one or several of glycerol kinase, glycerol-phosphate oxidase or peroxidase.

9. The use according to claim 7, wherein the colour development substrate is one or more of 3,3,5,5 - tetramethyl benzidine, 3,3,5,5 - tetramethyl benzidine hydrochloride, 4, 3,5-dichloro-2-hydroxybenzene sulfonic acid sodium, N, N - dimethyl aniline, N, N - dimethyl aniline dihydrochloride, N- (2 - hydroxy - 3 - sulfonated propyl) - 3,5 - dimethoxy aniline sodium, and 2,4,6-three bromine - 3-hydroxy benzoic acid.

10. The use according to claim 7, wherein the reagent further comprises a buffer reagent and a stabilizing reagent.

11. The use according to claim 10, wherein the stabilizing reagent is one or several of bovine serum albumin, polyethylene glycol 2000, polyvinyl pyrrolidone, or ethyl cellulose.

12. The use according to any of claims 1 to 11, wherein the sample is saliva, blood and urine.

13. The use according to claim 12, wherein the sample is saliva.

## Patentansprüche

1. Verwendung einer Vorrichtung zum Unterscheiden, ob ein Testsubjekt eine E-Zigarette raucht oder eine Zigarette (d. h. eine traditionelle Zigarette) raucht, **dadurch gekennzeichnet, dass**
die Vorrichtung einen Teststreifen, der dazu ausgelegt ist, Nikotin in einer Probe nachzuweisen; und einen Teststreifen umfasst, der dazu ausgelegt ist, Glycerin in der Probe nachzuweisen;
wobei, falls der Teststreifen zum Nachweisen von Nikotin positiv ist und der Teststreifen zum Nachweisen von Glycerin negativ ist, dies bedeutet, dass das Testsubjekt Zigaretten raucht;
wobei, falls der Teststreifen zum Nachweisen von Nikotin positiv ist und der Teststreifen zum Nachweisen von Glycerin positiv ist, dies bedeutet, dass das Testsubjekt sowohl Zigaretten als auch E-Zigaretten raucht;
wobei, falls der Teststreifen zum Nachweisen von Nikotin negativ ist und der Teststreifen zum Nachweisen von Glycerin positiv ist, dies bedeutet, dass das Testsubjekt E-Zigaretten raucht, wobei die Probe zum Nachweisen des Nikotins und des Glycerins verwendet wird.

2. Verwendung nach Anspruch 1, wobei der Teststreifen, der dazu ausgelegt ist, das Nikotin nachzuweisen, ein Lateral-Flow-Teststreifen ist.

3. Verwendung nach Anspruch 2, wobei der Lateral-Flow-Teststreifen das Nikotin durch Immunassay nachweist.

4. Verwendung nach Anspruch 3, wobei der Teststreifen Antikörper und Antigenanaloga zum Nachweisen von Cotinin und 3-Hvdroxycotinin umfasst.

5. Verwendung nach Anspruch 4, wobei der Antikörper mit einer Markierungssubstanz kombiniert ist und die Antikörper-Markierungssubstanz entfernt werden kann.

6. Verwendung nach Anspruch 5, wobei der Teststreifen Cotinin mit BSA, die am Testreifen fixiert sind, umfasst.

7. Verwendung nach Anspruch 1, wobei der Teststreifen, der dazu ausgelegt ist, Glycerin nachzuweisen, ein Reagenz zum Nachweisen des Glycerins umfasst und das Reagenz ein Farbentwicklungssubstrat und ein Enzym umfasst.

8. Verwendung nach Anspruch 7, wobei es sich bei dem Enzym um eines oder mehrere von Glycerinkinase, Glycerinphosphatoxidase oder -peroxidase handelt.

9. Verwendung nach Anspruch 7, wobei es sich bei dem Farbentwicklungssubstrat um eines oder mehrere von 3,3,5,5-Tetramethylbenzidin, 3,3,5,5-Tetramethylbenzidinhydrochlorid, 4,3,5-Dichlor-2-hydroxybenzolsulfonsäure-Natrium, N,N-Dimethylanilin, N,N-Dimethylanilindihydrochlorid, N-(2-Hydroxy-3-propylsulfonat)-3,5-dimethoxyanilin-Natrium und 2,4,6-drei-Brom-3-hydroxybenzoesäure handelt.

10. Verwendung nach Anspruch 7, wobei das Reagenz ferner ein Pufferreagenz und ein Stabilisierungsreagenz umfasst.

11. Verwendung nach Anspruch 10, wobei es sich bei dem Stabilisierungsreagenz um eines oder mehrere von Rinderserumalbumin, Polyethylenglycol 2000, Polyvinylpyrrolidon oder Ethylcellulose handelt.

12. Verwendung nach einem der Ansprüche 1 bis 11, wobei es sich bei der Probe um Speichel, Blut oder Urin handelt.

13. Verwendung nach Anspruch 12, wobei es sich bei der Probe uns Speichel handelt.

## Revendications

1. Utilisation d'un dispositif pour distinguer entre un sujet à l'essai qui fume une cigarette électronique et qui fume une cigarette (c'est-à-dire une cigarette traditionnelle), **caractérisée en ce que**,
le dispositif comprend une bandelette réactive adaptée à la détection de nicotine dans un échantillon ; et une bandelette réactive adaptée à la détection de glycérine dans l'échantillon ;
si la bandelette réactive de détection de nicotine est positive et que la bandelette réactive de détection de glycérine est négative, cela signifie que le sujet à l'essai fume des cigarettes ;
si la bandelette réactive de détection de la nicotine est positive et que la bandelette réactive de détection de glycérine est positive, cela signifie que le sujet à l'essai fume à la fois des cigarettes et des cigarettes électroniques ;
si la bandelette réactive de détection de nicotine est négative et que la bandelette réactive de détection de glycérine est positive, cela signifie que le sujet à l'essai fume des cigarettes électroniques, dans laquelle l'échantillon est utilisé pour la détection de la nicotine et de la glycérine.

2. Utilisation selon la revendication 1, dans laquelle la bandelette réactive adaptée à la détection de la nicotine est une bandelette réactive à flux latéral.

3. Utilisation selon la revendication 2, dans laquelle la bandelette réactive à flux latéral détecte la nicotine par immunodosage.

4. Utilisation selon la revendication 3, dans laquelle la bandelette réactive comprend des anticorps et des analogues d'antigène pour la détection de cotinine et de 3-hydroxycotinine.

5. Utilisation selon la revendication 4, dans laquelle l'anticorps est combiné à une substance de marquage et la substance de marquage d'anticorps peut être éliminée.

6. Utilisation selon la revendication 5, dans laquelle la bandelette réactive comprend de la cotinine avec de l'ASB fixée à la bandelette réactive.

7. Utilisation selon la revendication 1, dans laquelle la bandelette réactive adaptée à la détection de la glycérine comprend un réactif pour détecter la glycérine, et le réactif comprend un substrat de révélation de couleur et une enzyme.

8. Utilisation selon la revendication 7, dans laquelle l'enzyme est un ou plusieurs parmi la glycérol kinase, la glycérol-phosphate oxydase ou la peroxydase.

9. Utilisation selon la revendication 7, dans laquelle le substrat de révélation de couleur est un ou plusieurs parmi la 3,3,5,5-tétraméthyl benzidine, le chlorhydrate de 3,3,5,5-tétraméthyl benzidine, l'acide 4,3,5-dichloro-2-hydroxybenzène sulfonique sodique, la N,N-diméthyl aniline, le dichlorhydrate de N,N-diméthyl aniline, la N-(2-hydroxy-3-sulfonate propyl)-3,5-diméthoxy aniline sodique, et l'acide 2,4,6-tribromo-3-hydroxy benzoïque.

10. Utilisation selon la revendication 7, dans laquelle le réactif comprend en outre un réactif tampon et un réactif stabilisateur.

11. Utilisation selon la revendication 10, dans laquelle le réactif stabilisateur est un ou plusieurs parmi l'albumine de sérum bovin, le polyéthylène glycol 2000, la polyvinyl pyrrolidone ou l'éthyl cellulose.

12. Utilisation selon l'une quelconque des revendications 1 à 11, dans laquelle l'échantillon est de la salive, du sang et de l'urine.

13. Utilisation selon la revendication 12, dans laquelle l'échantillon est de la salive.
